(19) **Europäisches Patentamt**

**European Patent Office**

**Office européen des brevets**

(11) Veröffentlichungsnummer: **0 159 036 B1**

(12) **EUROPÄISCHE PATENTSCHRIFT**

(45) Veröffentlichungstag der Patentschrift: **05.08.92**

(21) Anmeldenummer: **85104712.6**

(22) Anmeldetag: **18.04.85**

(51) Int. Cl.⁵: **A61F 2/30**

(54) **Beschichtungsmasse und Verankerungsteil für Implantate.**

(30) Priorität: **19.04.84 DE 3414924**

(43) Veröffentlichungstag der Anmeldung:
**23.10.85 Patentblatt 85/43**

(45) Bekanntmachung des Hinweises auf die
Patenterteilung:
**05.08.92 Patentblatt 92/32**

(84) Benannte Vertragsstaaten:
**AT BE CH DE FR GB IT LI LU NL SE**

(56) Entgegenhaltungen:
**EP-A- 0 006 414        EP-A- 0 016 906
CH-A- 611 794           CH-A- 643 732
DE-A- 2 502 884         FR-A- 2 350 826
GB-A- 2 032 777**

(73) Patentinhaber: **Draenert, Klaus,
Dr.med.Dr.med.habil.
Gabriel-Max-Strasse 3
W-8000 München 90(DE)**

(72) Erfinder: **Draenert, Klaus,
Dr.med.Dr.med.habil.
Gabriel-Max-Strasse 3
W-8000 München 90(DE)**

(74) Vertreter: **Vossius & Partner
Siebertstrasse 4 P.O. Box 86 07 67
W-8000 München 86(DE)**

EP 0 159 036 B1

**Beschreibung**

Die Erfindung betrifft eine Beschichtungsmasse für chirurgische Implantate aus einem resorbierbaren Bindemittel und einem resorbierbarem Füllstoff aus hochporösen Kugelpartikeln aus einer festen Calciumverbindung. Die Erfindung betrifft ferner ein Verankerungsteil für chirurgische Implantate mit ganz oder teilweise beschichteter Oberfläche, wobei die Beschichtung aus der genannten Beschichtungsmasse besteht. Die beschichtete Oberfläche des Verankerungsteil ermöglicht wegen ihrer charakteristischen Ultrastruktur in besonderer Weise den Einwuchs des Knochens in das Implantat.

Eine künstliche Gelenkkomponente wird gewöhnlich mit einem Stift oder Schaft (Verankerungsteil) im Knochen verankert; vgl. J. Bone Joint Surg. Vol. 21, S. 269 - 288.

Verschiedene Verankerungstechniken sind in den US-Patentschriften 2,934,065 und 2,718,288 sowie in der französischen Patentschrift 1,278,359 beschrieben.

Ein Hüftgelenk wird z.B. häufig dadurch ersetzt, daß ein metallischer Vollschaft in die Knochenmarkhöhle versenkt und dort mit einem Zweikomponenten-Kunststoff verankert wird; vgl. J. Bone Joint Surg. Vol. 42 B, S. 28 - 30.

Resorbierbare chirurgische Materialien sind aus der GB-A-2 032 777 bekannt.

In der EP-A-0 016 906 sind Implantationsmaterialien auf der Basis von Kunststoffen offenbart. Die Verträglichkeit und die biomechanische Festigkeit dieser Verankerungs-Kunststoffe ist unbefriedigend, weil die als Bindemittel dienenden Kunststoffe Anteile an das umliegende Gewebe abgeben, die im Organismus schädliche Nebenreaktionen auslösen können. Außerdem kann es durch die beim Aushärten des Kunststoff-Klebers abfließende Reaktionswärme zu einer Gewebeschädigung kommen. Diese und andere Vorgänge bewirken eine Lockerung der Prothese. Es wurde deshalb auch versucht, zementlose Verankerungssysteme zu schaffen, um damit Prothesenkomponenten ohne die sogenannten Knochenzemente zu verankern; vgl. US-PS 3,605,123.

Dabei ist es in der Regel notwendig, die Oberfläche des Prothesenschaftes zu vergrößern, z.B. durch Anbringen von wellenartigen Oberflächengestaltungen, Sägezähnen usw; vgl. z.B. DE-PS 837 294.

In der Folge wurde versucht, die Oberfläche des Prothesenschaftes in verschiedenen Modifikationen zu strukturieren, um die Oberfläche zu vergrößern und den Knocheneinwuchs zu ermöglichen. So geht aus der DE-OS 21 27 843 eine poröse Metallbeschichtung hervor, die mit dem Basiskörper desselben Metalles fest verbunden ist. Bei all diesen Beschichtungen wurde festgestellt, daß ein knöcherner Einwuchs nur unter bestimmten Bedingungen erfolgen kann. Reproduzierbare, auf alle Patienten übertragbare Ergebnisse wurden mit derartigen Prothesenoberflächen jedoch nicht erreicht. Es muß angenommen werden, daß für die Sicherung des knöchernen Einwuchses und die prognostisch erfaßbare Verankerung auch andere Faktoren verantwortlich sind.

Bei der Prüfung der Frage, inwieweit die einzelne knochenbildende Zelle (Osteoblast) bzw. der Zellverband (Osteoblastenlayer) durch verschiedene morphologische oder sich chemisch unterscheidende Strukturen oder Stoffe zur Knochenbildung bzw. zum Aufbau bestimmter Knochenbälkcheninduziert werden können, hat es sich gezeigt, daß bezüglich der knochenbildenden Zelle eine bestimmte morphologische Struktur im Verankerungsteil die Knochenbildung stark induziert, während es im Hinblick auf den Zellverband eine andere bestimmte morphologische Struktur ist, die zur verstärkten Bildung eines tragenden Knochenbälkchens führt. Andererseits muß man die im Hinblick auf die zur Erzielung optimaler statischer Ergebnisse notwendigen bestimmten Ausgestaltungen (Topographie) der Prothesenverankerung bei der Gesamtkonstruktion berücksichtigen, wobei man die gesamte Beanspruchung und den Bewegungsablauf eines Gelenkes im Hinblick auf das Prothesendesign, das für die Krafteinleitung entscheidend ist, ebenfalls zu berücksichtigen hat.

Aus diesen aufgefundenen Kriterien ergeben sich vier Dimensionen der Strukturierung von Implantaten, die nachstehend als Struktur I. - IV. Ordnung bezeichnet werden.

Die Struktur erster Ordnung entspricht nach dieser Definition dem äußeren Prothesendesign, d.h. der Ausgestaltung des Verankerungsteils. Die Struktur zweiter Ordnung stellt bereits eine Betrachtung der Oberfläche (Topographie) dar. Beispielsweise werden mit Struktur II besondere Oberflächengestaltungen, wie eine wellenartige Oberflächengestaltung oder sägezähnartige Gestaltung des Prothesenschaftes bezeichnet. Diese Oberflächengestaltungen gemäß Struktur II sollen den Vorgang einer mechanischen Verankerung unterstützen. Die Struktur dritter Ordnung stellt nach der vorliegenden Definition die Mikrostruktur der Oberfläche dar. Sie umfaßt beispielsweise Oberflächenausgestaltungen (z.B. kleine Kugeln) bis in den mm-Bereich. Schließlich wird als Struktur IV. Ordnung die für die vorliegende Erfindung wesentliche Ultrastruktur (bis zu einer Größenordnung von 20 μm) bezeichnet.

Eine Ausgestaltung der vorstehend erwähnten Strukturen I.- III. Ordnung ist in der DE-PS 27 30 004 beschrieben. Aus der Druckschrift geht der Aufbau des Implantats vom Design der Prothese (Struktur erster Ordnung) bis zur Mikrostruktur (Struktur dritter Ordnung) hervor, die z.B. durch eine Kugelbeschichtung gelöst wird. Die Vorsprünge auf der Oberfläche des Basiskörpers sollen eine bessere Vernetzung des Knochengewebes in den Zwischenräumen zwischen den Vorsprüngen und somit eine widerstandsfähige Verankerung des Knochengewebes, vorzugsweise ohne Bindemittel, bewirken. Die Oberflächenschicht dieses bekannten Implantats weist jedoch den Nachteil auf, daß sie nicht resorbierbar ist, wodurch sich keine gute Haftung der Knochenzellen an den Basiskörper ergibt. Auch bioaktive und die chemotaktische Wirkung (Knocheninduktion) sind bei nicht resorbierbaren Oberflächenschichten unbefriedigend. Schließlich können der Oberflächenschicht gemäß DE-PS 27 30 004 keine Zusätze, wie Hämostyptika, knochenbildende Substanzen, Antibiotika, gefäßwirksame Substanzen und knochenwirksame Hormone einverleibt werden.

In der DE-PS 26 20 907 wird eine Beschichtung aus nicht resorbierbarem Kunststoff mit kugelförmigen Füllstoffen beschrieben, die aus größtenteils resorbierbarem Calciumphosphat bestehen. Die nicht resorbierbare Kunststoffmatrix hat den Nachteil, daß der Kunststoff durch die im Interface auftretenden Scherkräfte zerrieben wird und das Abriebmaterial nicht resorbiert werden kann, was zu Entzündungen führt und die Lockerung der Prothese zur Folge haben kann. Ein weiterer wesentlicher Nachteil der Beschichtung gemäß DE-PS 26 20 907 liegt darin, daß sich die als resorbierbar bezeichneten Keramikpartikel mit dem nicht resorbierbaren Kunststoff vollsaugen, was zu einer weiteren Verringerung der Resorbierbarkeit der gesamten Beschichtung führt. Deshalb kann der die Prothesenverankerung umgebende Knochen nicht tief und schnell an den Basiskörper (Stütze) heranwachsen, was einen Festigkeitsverlust zur Folge hat.

Der Erfindung liegt die Aufgabe zugrunde, eine Beschichtungsmasse für chirurgische Implantate zu schaffen, die vollständig resorbierbar ist und tiefes und schnelles Einwachsen des Knochens ermöglicht. Eine weitere Aufgabe der Erfindung ist die Bereitstellung eines Verankerungsteils für chirurgische Implantate mit ganz oder teilweise beschichteter Oberfläche, bei dem die Beschichtung vollständig resorbierbar ist, das tiefes und schnelles Einwachsen des Knochens bis an den Basiskörper des Implantats ermöglicht und das einen festen Sitz des Implantats gewährleistet. Diese Aufgaben werden durch die Erfindung gelöst.

Gegenstand der Erfindung ist somit eine Beschichtungsmasse für chirurgische Implantate, bestehend aus einem resorbierbaren Füllstoff auf der Basis fester Calciumverbindungen und einem resorbierbaren Bindemittel (Matrix), das dadurch gekennzeichnet ist, daß der Füllstoff aus hochporösen Kugelpartikeln mit einem Durchmesser von 10 bis 200 $\mu$m und einem Porenvolumen von 25 bis 80 % besteht.

Gegenstand der Erfindung ist ferner ein Verankerungsteil für chirurgische Implantate mit ganz oder teilweise beschichteter Oberfläche, wobei die Beschichtung aus einem resorbierbaren Füllstoff auf der Basis fester Calciumverbindungen und einem Bindemittel (Matrix) besteht, das dadurch gekennzeichnet ist, daß auch das Bindemittel resorbierbar ist und der Füllstoff aus hochporösen Kugelpartikeln mit einem Durchmesser von 10 bis 200 $\mu$m und einem Porenvolumen von 25 bis 80 % besteht.

In einer besonderen Ausführungsform kann die Beschichtungsmasse auf dem Verankerungsteil eine zusätzliche Armierung aufweisen. Diese Armierung kann aus Fasern bestimmter Länge und Dicke oder aus einem geschlossenen Netz in Form eines Prothesenstrumpfes bestehen.

Die Erfindung beruht auf der Erkenntnis, daß es im wesentlichen zwei Faktoren gibt, die zum einen den gewünschten Knocheneinwuchs induzieren und zum anderen die Morphologie einer tragenden oder nicht tragenden Knochenstruktur und die damit zusammenhängende Möglichkeit, auftretende Belastungen störungsfrei aufzunehmen, bestimmen können. Es ist dies einerseits die Morphologie der Oberflächenstruktur im Verankerungsteil und andererseits die chemische Zusammensetzung seiner Oberfläche.

Es hat sich gezeigt, daß Kugeln oder Kugelteilflächen mit einem Durchmesser von etwa 10 bis etwa 200 $\mu$m, vorzugsweise von etwa 15 bis etwa 50 $\mu$m, insbesondere von etwa 20 $\mu$m, die optimal strukturierte Oberfläche (Ultrastruktur vierter Ordnung) darstellen, die ein Osteoblast als seine Basis erkennen kann.

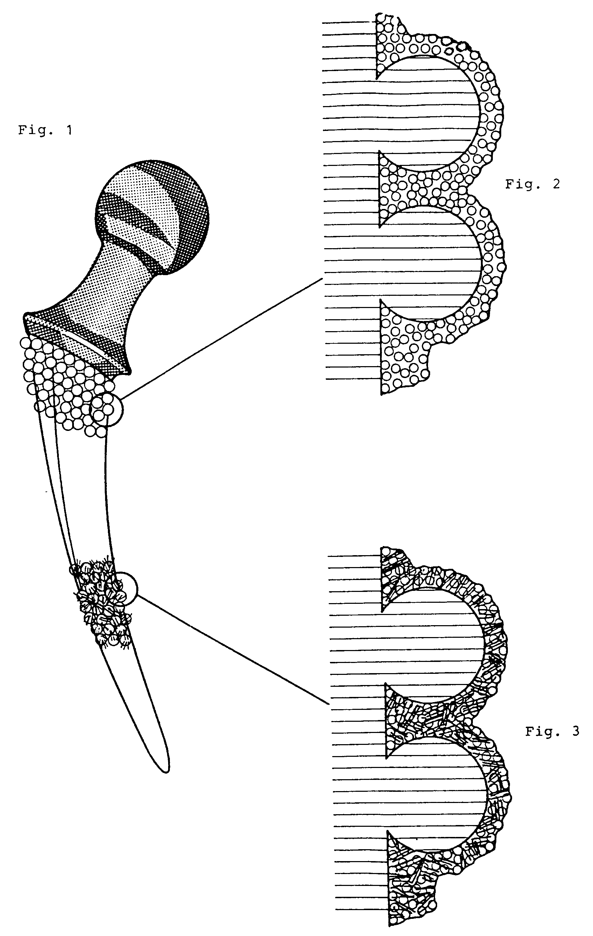

Figur 1 zeigt die Femoralkompenente einer Hüftgelenks-Endoprothese mit zwei Ausführungsformen der erfindungsgemäßen Oberflächengestaltung des Verankerungsteils.

Figur 2 zeigt im Längsschnitt eine Implantatoberfläche mit zwei kugelförmigen Elementarkörpern (Struktur III.Ordnung) und einer Beschichtung aus einer Matrix und kugelförmigem Füllstoff.

Figur 3 zeigt einen ähnlichen Gegenstand wie Figur 2, wobei jedoch die Beschichtung eine zusätzliche Armierung durch Fasern aufweist. Die Fasern sind in dieser Abbildung nicht maßstabsgerecht gezeichnet. Sie sind in Wirklichkeit länger, wie aus Abbildung 1 ersichtlich.

Figur 4 zeigt schematisch zwei Kugelpartikel (Füllstoff der Beschichtungsmasse der Erfindung) die die Basis eines Osteoblasten bilden.

Figur 5 zeigt einen ähnlichen Gegenstand wie Figur 2, wobei jedoch die Beschichtung eine zusätzliche Armierung aus einer Kugelkette aufweist.

Figur 6a zeigt einen ähnlichen Gegenstand wie Figur 5, wobei jedoch die Armierung aus einem mehrschichtigen Prothesenstrumpf besteht.

Figur 6b zeigt eine Ausschnittsvergrößerung von Figur 6a.

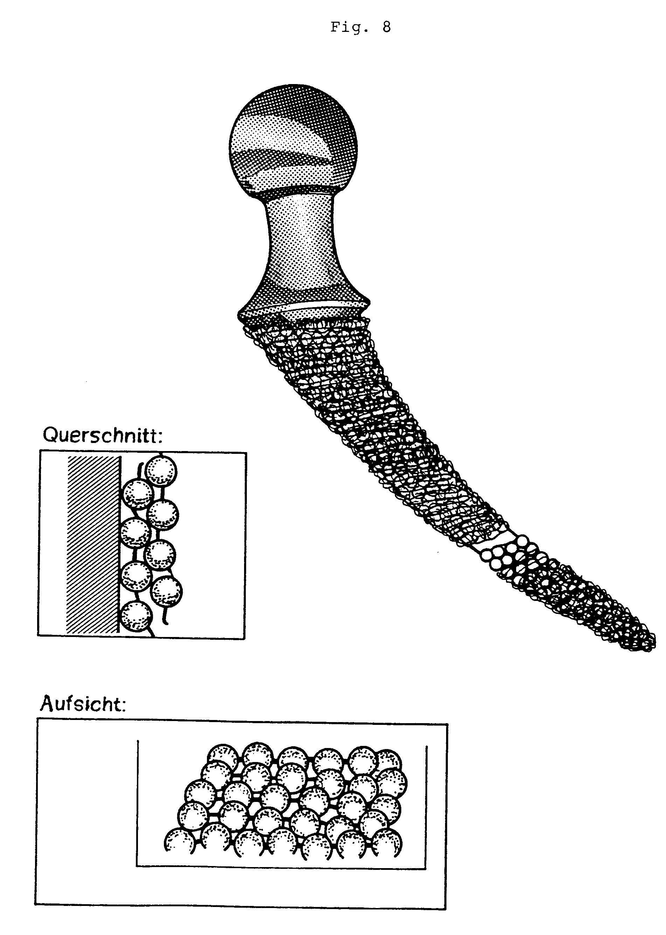

Figur 7 zeigt den Verankerungsteil einer in Figur 1 beschriebenen Prothese, jedoch mit einer Strumpfarmierung der organischen Beschichtung.

Figur 8 zeigt den Verankerungsteil einer in Figur 1 beschriebenen Prothese, jedoch mit einer Strumpfarmierung der organischen Beschichtung, aufgetragen auf eine aus einem Kugelkettenstrumpf bestehende Beschichtung. Figur 8 sind auch der Querschnitt und die Aufsicht der genannten Kugelketten zu entnehmen.

Die Beschichtungsmasse der Erfindung, die zur Herstellung der erfindungsgemäßen Verankerungsteile verwendet wird, weist die Besonderheit auf, daß sie vollständig resorbierbar ist (sowohl Matrix als auch Füller). Sie zeigt weiterhin charakteristische Eigenschaften in der vorstehend erläuterten Oberflächenstruktur vierter Ordnung (Ultrastruktur).

Der Füllstoff besteht aus hochporösen Kugelpartikeln mit einem Durchmesser von 10 bis 200 $\mu$m, vorzugsweise 15 bis 50 $\mu$m, besonders bevorzugt 15 bis 30 $\mu$m, optimal etwa 20 $\mu$m. Diese Kugelpartikel weisen ein Porenvolumen von 25 bis 80, vorzugsweise 50 bis etwa 80, besonders bevorzugt etwa 50 bis 65 % auf.

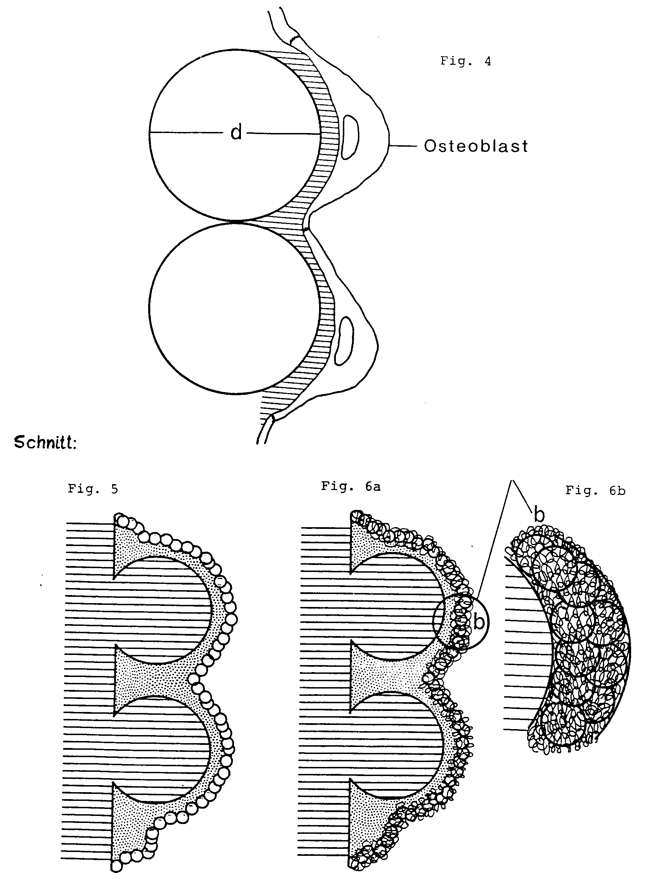

Aufgrund der erfindungsgemäßen Ultrastruktur wird die einzelne knochenbildende Zelle (Osteoblast ) bzw. der Zellverband (Osteoblastenlayer) zum Aufbau bestimmter Knochenbälkchen angeregt. Weiterhin wird aufgrund der Struktur des erfindungsgemäßen Knochenersatzwerkstoffs die einzelne Knochenzelle angeregt. Dadurch erfolgt ein sehr rascher Einwuchs des Knochens in den Ersatzwerkstoff. In Figur 4 bezeichnet d den Durchmesser der Kugelteilchen, der vorzugsweise etwa 20 $\mu$m beträgt, und in der Größenordnung eines Osteoblasten liegt.

Die den Füllstoff darstellenden hochporösen Kugelpartikel bestehen vorzugsweise aus Tricalciumphosphat oder Apatit ("Hydroxylapatit"). Ihre Poren können mit einem resorbierbaren, körperverträglichen Stoff gefüllt sein. Dieser Stoff ist vorzugsweise das verwendete Bindemittel. Spezielle Beispiele für den resorbierbaren, körperverträglichen Stoff bzw. das Bindemittel sind Polyaminosäuren, Polylactate, Polyglykolate, Cokondensate dieser Stoffe, Gelatine, Kollagen und Calciumverbindungen. Die Füllkörper sind vorzugsweise härter als das Bindemittel.

Weiterhin wurde erfindungsgemäß gefunden, daß eine organische Matrix zu einer sehr viel schnelleren Ausbildung einer Osteoblastenschicht führt als eine Metall- oder Keramikoberfläche. Es wurde auch gefunden, daß die Keramikoberfläche zwar besser kolonisiert wird als eine Metalloberfläche; die Oberfläche von gesintertem Apatit ist aber nochmals einer rascheren Zellkolonisation zugänglich als eine Keramikoberfläche. Es wurde auch gefunden, daß eine Kollagenmatrix noch rascher durch Zellen kolonisiert wird (besiedelt wird) als eine reine Keramik- oder eine reine Apatitoberfläche. Die besten Kolonisationsraten werden erzielt, wenn die organische Matrix (z.B. Kollagen) mit kleinen Kügelchen aus Apatit bestückt ist. Diese Beschichtungsmasse stellt eine im Sinne dieser Erfindung bevorzugte Ausführungsform der Struktur IV. Ordnung (Ultrastruktur) dar.

Die erfindungsgemäße Beschichtungsmasse ist vollständig resorbierbar. Eine vollständig resorbierbare Matrix hat gegenüber einer nicht resorbierbaren Matrix ganz wesentliche Vorteile. Sie wird in den Zonen der Beanspruchung sehr schnell resorbiert und durch Knochen ersetzt. Durch eine Kugelanordnung III. Ordnung (vgl. Fig. 2) kann in Verbindung mit einem dünnen Überzug aus der Beschichtungsmasse der Erfindung der über den Kugeln angebracht wird, in sehr kurzer Zeit eine geschlossene Osteoblastenschicht (Osteoblastenlayer) erhalten werden. Diese Osteoblastenschicht ist in der Lage, einen lamellären, stark beanspruchbaren Knochen zu bilden. Durch die Dichte der Kugelpackung und ihre konkrete Größe wird somit erreicht, daß der Anordnung von knochenbildenden Zellen primär eine bestimmte Struktur vorgegeben wird, so daß eine Umordnung der Zellen und der sich bildenden Knochenbälkchen nicht erforderlich wird. Dadurch wird die Zeit der Resorption und Knochenneubildung (Remodelling) eingespart.

In einer Ausführungsform der Erfindung kann die Schicht aus der Beschichtungsmasse auf dem Verankerungsteil eine zusätzliche Armierung aufweisen. Diese Armierung kann z.B. aus verschieden langen Fasern bestehen, die eine Dicke von 100 bis 300, insbesondere etwa 200 $\mu$m aufweisen. Die Fasern sind vorzugsweise mehr als 2 bis 15 mm lang, besonders bevorzugt mindestens 3 mm und höchstens 10 mm lang, mit einer optimalen Länge von etwa 4 bis 5 mm.

Beispiele für Stoffe, aus denen die faserförmigen Füllstoffe bestehen können, sind Polyaminosäuren, Polylactate, Polyglykolate, Cokondensate dieser Stoffe, Gelatine, Kollagen, Kohlenstoff oder Catgut. Der Menge der Fasern zur Armierung kann etwa 5 bis 15, vorzugsweise etwa 10 Gew.-%, bezogen auf die Menge derhochporösen Kugelpartikel betragen.

In einer weiteren Ausführungsform der Erfindung ist die zusätzliche Armierung ein geschlossenes Netz in Form eines Prothesenstrumpfes.

Der Prothesenstrumpf kann auch aus Kugelketten bestehen, in denen die Kugeln einen Durchmesser von 15 bis 50 $\mu$m, insbesondere etwa 20

μm aufweisen, wobei die Kugeln so dicht gelagert sind, daß sie in einem dreidimensionalen Verbund aneinanderstoßen. Die Netze können auch als mehrschichtiger Strumpf angeordnet sein.

Die Herstellung der Beschichtungsmasse und des Verankerungsteils der Erfindung wird nachstehend am Beispiel von Kollagen als vollständig resorbierbares Bindemittel erläutert. Nach einem üblichen Verfahren wird aus tierischen Knochen eine Kollagenmasse hergestellt, die wie ein Leim mit kugelförmigem Apatit oder TCP versetzt werden kann (der Ausdruck "Apatit" bedeutet in der Erfindung vorzugsweise "Hydroxylapatit", "TCP" bedeutet "Tricalciumphosphat"). Durch Anwendung von Infraschall, Schüttelrührwerk und/oder anderen Rührwerken kann eine dichte Kugelpackung erzeugt werden. Mit dieser Beschichtungsmasse wird dann ein Implantat imprägniert. Dabei wird ein Aufbau erhalten, in dem die erfindungsgemäße Struktur IV. Ordnung einen Überzug auf der Struktur III. Ordnung bildet; vgl. Figur 2.

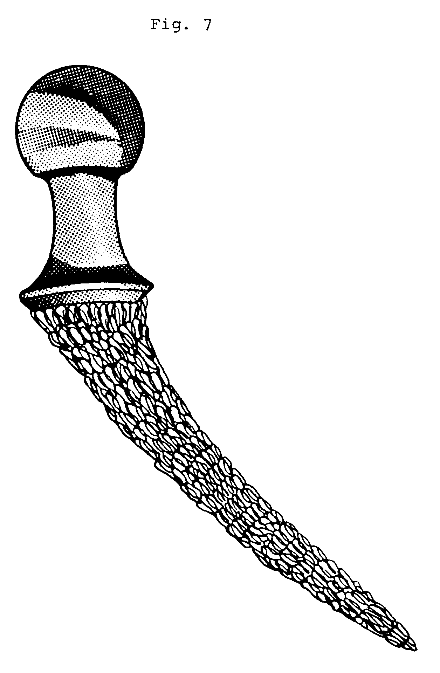

Ein Beispiel für die Herstellung eines Prothesenstrumpfes als zusätzliche Armierung wird nachstehend gegeben: Aus der vorstehend beschriebenen und mit Apatit bzw. TCP bestückten Kollagenmasse werden Kugeln der optimalen Größe von 200 bis 3000 μm hergestellt und auf einem resorbierbaren Faden zu Kugelketten aufgereiht. Diese Kugelketten werden in einer Rundstrickmaschine zu fortlaufenden Strümpfen verarbeitet, wobei ein Kugelkettennetzwerk entsteht. Von diesem Kugelkettennetzverband können durch Ineinanderstülpen alle Formen von mehrlagigen Prothesenstrümpfen hergestellt werden. Kugelkettennetzwerke oder Prothesenstrümpfe können zur zusätzlichen Armierung der Verankerungsteile dienen; vgl. Fig. 5 und 6.

Die Beschichtungsmasse der Erfindung kann auf einen Prothesenstift oder -schaft aufgebracht werden, wobei der Überzug während des Trocknens auf dem Verankerungsteil der Prothese aufschrumpft und zu einem geschlossenen Kontakt zwischen Matrix, Füllstoff und dem Basismaterial führt. Eine derart beschichtete Prothese kann nun ohne bindenden Knochenzement in den Knochen eingesetzt werden. Bei entsprechender Abstützung entstehen in kurzer Zeit geschlossene knochenbildende Lagen auf der Oberfläche, die die Prothesenkomponente stabil knöchern abstützen können. Dabei bilden sich ausgesprochene Stütztrabekel aus, neben denen der Matrixüberzug resorbiert wird, so daß in der Folge der Knochen tief in die Oberflächenstruktur des Basiskörpers (Stützgerüst) einwachsen kann. Durch die induzierende Wirkung der kleinen, beispielsweise von Kollagen bedeckten Kugeln aus Apatit kommt es zu einem sehr viel schnelleren Knochenanbau und -einbau des Implantates, als dies bei herkömmlichen Beschichtungen oder nicht beschichteten Implantaten der Fall ist.

Die Induktion der Knochenbildung kann durch Hinzufügen chemischer Wirkstoffe in die Matrixsubstanz noch erhöht werden. Beispielsweise sind chemotaktisch wirksame Stoffe aus Knochengrundsubstanz und nekrotischen Knochen bekannt, die sogenannte "bone morphogenic" Proteine enthalten, welche eine besonders induzierende Wirkung auf die Knochenbildung ausüben. Die Zugabe solcher Zusätze ist bevorzugt.

Es hat sich gezeigt, daß die Induktion der Knochenbildung dann besonders günstig ist, wenn die als Füllstoff eingesetzten hochporösen Kugelpartikel härter sind als die umgebende Matrix, so daß sie eine mechanisch stimulierende Wirkung auf die knochenbildende Zelle ausüben. Eine solche Ausführungsform ist ebenfalls bevorzugt.

Ein weiterer Vorteil des erfindungsgemäßen Verankerungsteils liegt darin, daß er eine beträchtliche Bindemittelmasse enthält. Es hat sich gezeigt, daß diese eine recht beachtliche Kapazität mit guten Freisetzungsraten im Hinblick auf Wirkstoffzusätze aufweist. Es ist bekannt, daß Implantate, die der Körperabwehr aufgrund der fehlenden Vaskularisation nicht zugänglich sind, dadurch gefährdet sind, daß sie sehr leicht von Bakterien besiedelt werden. Dies kann dadurch verhindert werden, daß man der Beschichtungsmasse ein Antibiotikum zusetzt. Solche Antibiotikabeimengungen sind von den Knochenzementen her sehr gut erforscht. Die zementfreien Prothesen verfügten bislang nicht über diesen Schutz. Mit der Beschichtungsmasse aus resorbierbaren Stoffen ist es jedoch möglich, auch die zementfreien Prothesen und Implantate, gleich welcher Art, mit einem wirksamen Infektionsschutz prophylaktisch zu versorgen.

Die lokale Applikation anderer Medikamente, wie Hämostyptika, ist bei äußeren Behandlungen bekannt. Durch den Zusatz solcher Stoffe zur Beschichtungsmasse der Erfindung können solche Wirkstoffe auch dort wirksam werden, wo die Organe einer äußeren Behandlung nicht zugängig sind. Hämostyptika als Bestandteile des Bindemittels sorgen für eine sofortige Blutstillung im knöchernen Lager. Gefäßwirksame Substanzen, wie Noradrenalin oder eines seiner Abkömmlinge, führen über die Gefäßverengung ebenfalls zu einem blutstillenden Effekt. Dadurch wird die Durchtränkung der Grenzschicht mit Blut verhindert, wodurch die mechanische Festigkeit der Grenzzone länger erhalten bleibt. Ferner können, wie bereits erwähnt, knocheninduzierende, chemotaktisch wirksame Substanzen dem Bindemittel beigemengt werden. Diese führen zu einem schnelleren Beginn der Knochenneubildung. Mit Substanzen vom Typ des Calcitonin sind Hormonapplikationen lokal wirksam, die verhindern, daß der neugebildete Knochen wieder abgebaut wird. Das Beispiel erläutert die Erfindung.

Anwendungsbeispiel zur Herstellung eines Verankerungsteils mit einer vollständig resorbierbaren Beschichtung

In dem nachstehenden Beispiel weist die Beschichtung des Verankerungsteils für Implantate als Füllstoff hochporöses Tricalciumphosphat in Kugelform auf. Das Verankerungsteil ist durch einen resorbierbaren Fadenstrumpf zusätzlich armiert.

1.1 Zunächst wird mit einen 200 μm dicken und resorbierbaren Faden auf einer herkömmlichen Rundstrickmaschine (Fa. Dubier, Neuchatel) ein Gewirk gestrickt (im Sinne eines endlosen Strumpfes). Dieser Strumpf wird so über die Prothese gestülpt, daß er durch Drehung von 90° bis 180° in einer zweiten Schicht über den Verankerungsteil gezogen werden kann. Durch erneutes Verdrehen des Strumpfes über der Prothesenspitze wird nunmehr eine dritte Schicht aufgebracht. Auf diese Weise kann ein mehrschichtiger Strumpf auf den Prothesenschaft aufgezogen werden. Das Fadengewirke kann unter einer beliebigen Vorspannung auf den Prothesenschaft gehalten werden. Anschließend wird der mit den Strümpfen überzogene Prothesenschaft mit einer klebrigen und mit Tricalciumphosphatkügelchen (Durchmesser 15 bis 50 μm) versehenen Masse beschichtet. Die Beschichtung erfolgt durch Aufstreichen oder durch Eintauchen der Prothese. Die Masse wird sodann getrocknet und erhärtet. Sie bildet einen geschlossenen Mantel mit fein- und grobstrukturierter Oberfläche um den Verankerungsteil der Prothese oder eines Implantates.

1.2 Die resorbierbare Matrix kann in sehr einfacher Weise und wirtschaftlich dadurch hergestellt werden, daß Kollagen aus tierischem Knochen gewonnen wird und analog den bekannten Verfahren zur Herstellung von Knochenleim zu einer plastischen und klebrigen Masse verarbeitet wird. Die diesem Verfahren zugrundeliegenden chemischen Vorgänge sind bekannt. Sie bestehen im wesentlichen in der Fällung des Eiweißes, der Quellung der Kollagene und ihrer Demineralisierung. Die aus diesen bekannten Verfahren hervorgehenden Kollagensubstanzen haben keinerlei antigene Eigenschaften mehr und sind gut körperverträglich. Die auf diese Weise aus tierischen Knochen hergestellte Kollagenmasse wird im Anschluß daran getrocknet und zu Granulat verarbeitet. Das Granulat kann unter Zugabe einer physiologischen Elektrolytlösung und weiterhin unter Zugabe von hochporösen Tricalciumphosphatkügelchen in dem beanspruchten Größenbereich zu einer teigigen Masse verarbeitet werden. Diese wird durch Zugabe von Alkohol und Elektrolytlösung auf die gewünschte Viskosität eingestellt.

1.3 Die Beschichtung der Implantate kann z.B. nach dem Tauchverfahren erfolgen. Bei niedrigen bis mittleren Viskositäten der verwendeten Kollagenmasse wird das Implantat eingetaucht. Die klebrige Masse bleibt hierbei am Verankerungsteil der Prothese hängen und überzieht diese mit einem geschlossenen Mantel. Durch Schrumpfen der Matrix während des Trocknungsvorgangs entsteht eine abdruckmäßige Anpassung an das Verankerungsteil.

Ein weiteres Verfahren für die Beschichtung stellt die feine Zerstäubung des Matrixmantels oder eine Beschichtung mittels elektrostatischer Aufladung dar, wodurch es zu einem geschlossenen und sehr dünnen Belag auf der Prothese kommt. Diese Verfahren sind von ihrer Technik her aus den Lackierungsvorgängen der Autoindustrie bekannt.

Eine Beschichtung kann jedoch auch in der Weise erfolgen, daß die Matrix unter hohem Druck aufgepreßt wird.

Schließlich kann, insbesondere wenn es sich um Polyaminosäuren oder Polyglykolate handelt, die Beschichtung auch über einen Schmelzprozeß erfolgen.

1.4 Bei der Herstellung des erfindungsgemäß eingesetzten hochporösen Tricalciumphosphates in Form von Kugeln treten die folgenden chemischen Reaktionen auf:

$$2\ CaHPO_4\ +\ CaCo_3\ \rightarrow\ Ca_3P_2O_8\ +\ CO_2 + H_2O$$

Aus einer Pulvermischung von Calciumhydrogenphosphat und Calciumcarbonat in einem Verhältnis von 2:1 entsteht im wesentlichen Tricalciumphosphat, aus dem bei Temperaturen um 1500°C durch Sinterung und Pressen ein Tricalciumphosphat in der Alpha- bzw. Beta-Form entsteht. Durch schnelles Abkühlen kann ein sehr hochporöses Tricalciumphosphat in gesinterter Form erzeugt werden. Dieses weist eine Porösität von 25 bis 80 % auf, wie es erfindungsgemäß gefordert wird.

Diese Form des Tricalciumphosphats ist besonders schnell resorbierbar. Aus diesem Material können in der Kugelmühle die entsprechenden Kügelchen in der beanspruchten Größe erzeugt werden. Die gewünschten Größenfraktionen werden in an sich bekannter Weise durch Aussieben erhalten.

1.5 Tricalciumphosphatkügelchen können auch aus Pulver erzeugt werden. Dabei wird eine Mischung der resorbierbaren Matrix (z.B. Kollagenmatrix) mit pulverförmigem Tricalciumphosphat hergestellt. Beispielsweise werden 5 g Kollagen und 2 g Tricalciumphosphat als feines, im Handel erhältliches Pulver vermischt. Durch Trocknen dieses Breies entsteht nun eine feste Substanz, die wiederum durch Zerkleinerung, Bearbeitung in der Kugelmühle und Aussiebens zu feinen Tricalciumphosphat-Matrixkügelchen verarbeitet werden kann.

**Patentansprüche**

1.  Beschichtungsmasse für chirurgische Implantate, bestehend aus einem resorbierbaren Füllstoff auf der Basis fester Calciumverbindungen und einem resorbierbaren Bindemittel (Matrix), dadurch gekennzeichnet, daß der Füllstoff aus hochporösen Kugelpartikeln mit einem Durchmesser von 10 bis 200 μm und einem Porenvolumen von 25 bis 80% besteht.

2.  Beschichtungssmasse nach Anspruch 1, dadurch gekennzeichnet, daß die Kugelpartikel einen Durchmesser von 15 bis 50 μm, insbe sondere etwa 20 μm, und ein Porenvolumen von 50 bis 80, insbesondere 50 bis 65 % aufweisen.

3.  Beschichtungsmasse nach Anspruch 1 oder 2, dadurch gekennzeichnet, daß die Kugelpartikel aus hochporösem Tricalciumphosphat oder Apatit bestehen, dessen Poren mit einem resorbierbaren, körperverträglichen Stoff, z.B. dem Bindemittel der Beschichtungsmasse gefüllt sind.

4.  Beschichtungsmasse nach einem der Ansprüche 1 bis 3, dadurch gekennzeichnet, daß der resorbierbare körperverträgliche Stoff bzw. das Bindemittel eine Polyaminosäure, ein Polylactat, Polyglycolat, ein Cokondensat dieser Stoffe, Gelatine, Kollagen oder eine Calciumverbindung ist.

5.  Beschichtungsmasse nach Anspruch 1, dadurch gekennzeichnet, daß die Kugelpartikel härter als das Bindemittel sind.

6.  Beschichtungsmasse nach Anspruch 1, dadurch gekennzeichnet, das sie mindestens einen Zusatz aus der Gruppe Hämostyptika, knochenbildende Substanzen, Antibiotika, gefäßwirksame Substanzen und knochenwirksame Hormone enthält.

7.  Verankerungsteil für chirurgische Implantate mit ganz oder teilweise beschichteter Oberfläche, wobei die Beschichtung aus einem resorbierbaren Füllstoff auf der Basis fester Calciumverbindungen und einem Bindemittel (Matrix) besteht, dadurch gekennzeichnet, daß die Beschichtung aus einer Beschichtungsmasse nach einem der Ansprüche 1 bis 6 besteht.

8.  Verankerungsteil nach Anspruch 7, dadurch gekennzeichnet, daß die Beschichtung eine zusätzliche Armierung aufweist.

9.  Verankerungsteil nach Anspruch 8, dadurch gekennzeichnet, daß die Armierung aus verschieden langen Fasern mit einer Länge von 2 bis 15 mm und einer Dicke von 100 bis 300 μm besteht.

10.  Verankerungsteil nach Anspruch 9, dadurch gekennzeichnet, daß die Fasern aus einer Polyaminosäure, einem Polylactat, Polyglycolat, einem Cokondensat dieser Stoffe, Gelatine, Kollagen, Kohlenstoff oder Catgut bestehen.

11.  Verankerungsteil nach Anspruch 8, dadurch gekennzeichnet, daß die Armierung aus einem geschlossenen Netz in Form eines Prothesenstrumpfes besteht.

12.  Verankerungsteil nach Anspruch 11, dadurch gekennzeichnet, daß der Prothesenstrumpf aus Kugelketten besteht, deren Kugeln einen Durchmesser von 15 bis 50 μm aufweisen, wobei die Kugeln so dicht gelagert sind, daß sie in einem dreidimensionalen Verbund aneinanderstoßen.

**Claims**

1.  Coating composition for surgical implants, consisting of a resorbable filling material with solid calcium compounds as a basis and a resorbable binding agent (matrix), characterized in that the filling material consists of highly porous spherical particles having a diameter from 10 to 200 μm and a pore volume from 25 to 80%.

2.  Coating composition according to claim 1, characterized in that the spherical particles have a diameter from 15 to 50 μm, in particular about 20 μm, and a pore volume from 50 to 80%, in particular 50 to 65%.

3.  Coating composition according to claim 1 or 2, characterized in that the spherical particles consist of highly porous tricalcium phosphate or apatite, the pores of the coating composition being filled with a resorbable, biologically compatible material, e.g. the binding agent of the coating composition.

4.  Coating composition according to any of claims 1 to 3, characterized in that the resorbable, biologically compatible material and/or the binding agent is a polyamino acid, a polylactate, a polyglycolate, a cocondensate of these substances, gelatine, collagen or a calcium compound.

5.  Coating composition according to claim 1, characterized in that the spherical particles are harder than the binding agent.

6.  Coating composition according to claim 1, characterized in that it contains at least one admixture from the group of hemostyptics, osteogenic substances, antibiotics, vasoactive substances and hormones acting on bone.

7.  Anchorage component for surgical implants having a fully or partly coated surface, the coating consisting of a resorbable filling material with solid calcium as a basis and a binding agent (matrix), characterized in that the coating consists of a coating composition according to any of claims 1 to 6.

8.  Anchorage component according to claim 7, characterized in that the coating comprises an additional armament.

9.  Anchorage component according to claim 8, characterized in that the armament comprises fibers of differing lengths from 2 to 15 mm and a thickness of 100 to 300 $\mu$m.

10. Anchorage component according to claim 9, characterized in that the fibers consist of a polyamino acid, a polylactate, a polyglycolate, a co-condensate of these substances, gelatine, collagen, carbon or catgut.

11. Anchorage component according to claim 8, characterized in that the armament comprises a closed network in the form of a prosthetic stocking.

12. Anchorage component according to claim 11, characterized in that the prosthetic stocking is made of chains of spheres, the spheres of which have a diameter of 15 to 50 $\mu$m and wherein the spheres are packed so tightly that they are in contact with each other in a three-dimensional arrangement.

**Revendications**

1.  Matériau de revêtement pour implants chirurgicaux, constitué d'une matière de remplissage résorbable à base de composés de calcium solides et d'un liant résorbable (matrice), caractérisé en ce que la matière de remplissage est constituée de particules sphériques hautement poreuses ayant un diamètre de 10 à 200 $\mu$m et un volume poreux de 25 à 80 %.

2.  Matériau de revêtement suivant la revendication 1, caractérisé en ce que les particules sphériques présentent un diamètre de 15 à 50 $\mu$m, en particulier d'environ 20 $\mu$m, et un volume poreux de 50 à 80 %, en particulier de 50 à 65 %.

3.  Matériau de revêtement suivant l'une des revendications 1 et 2, caractérisé en ce que les particules sphériques sont constituées de phosphate tricalcique hautement poreux ou d'apatite dont les pores sont remplis d'une matière résorbable, compatible avec le corps, par exemple du liant du matériau de revêtement.

4.  Matériau de revêtement suivant l'une des revendications 1 à 3, caractérisé en ce que la matière résorbable compatible avec le corps ou respectivement le liant est un polyamino-acide, un polylactate, un polyglycolate, un produit de cocondensation de ces substances, de la gélatine, du collagène ou un composé de calcium.

5.  Matériau de revêtement suivant la revendication 1, caractérisé en ce que les particules sphériques sont plus dures que le liant.

6.  Matériau de revêtement suivant la revendication 1, caractérisé en ce qu'il contient au moins une addition choisie parmi le groupe des hémostyptiques, des substances ostéogènes, des antibiotiques, des substances actives sur les vaisseaux et des hormones actives sur les os.

7.  Pièce d'ancrage pour implants chirurgicaux présentant une surface totalement ou partiellement revêtue, le revêtement étant constitué d'une matière de remplissage résorbable à base de composés de calcium solides et d'un liant (matrice), caractérisée en ce que le revêtement est constitué d'un matériau de revêtement suivant l'une des revendications 1 à 6.

8.  Pièce d'ancrage suivant la revendication 7, caractérisée en ce que le revêtement présente une armature supplémentaire.

9.  Pièce d'ancrage suivant la revendication 8, caractérisée en ce que l'armature est constituée de fibres de différentes longueurs ayant une longueur de 2 à 15 mm et une grosseur de 100 à 300 μm.

10. Pièce d'ancrage suivant la revendication 9, caractérisée en ce que les fibres sont constituées d'un polyaminoacide, d'un polylactate, d'un polyglycolate, d'un produit de cocondensation de ces substances, de gélatine, de collagène, de carbone ou de catgut.

11. Pièce d'ancrage suivant la revendication 8, caractérisée en ce que l'armature est constituée d'un filet fermé sous la forme d'une chaussette de prothèse.

12. Pièce d'ancrage suivant la revendication 11, caractérisée en ce que la chaussette de prothèse est constituée de chaînes de sphères dont les sphères présentent un diamètre de 15 à 50 μm, les sphères étant supportées de manière suffisamment dense pour qu'elles se touchent l'une l'autre dans un assemblage tri-dimensionnel.

Fig. 1

Fig. 2

Fig. 3

Fig. 4

d

Osteoblast

Schnitt:

Fig. 5

Fig. 6a

Fig. 6b

b

Fig. 7

Fig. 8

Querschnitt:

Aufsicht: